# EUROPÄISCHE PATENTSCHRIFT

(11) **EP 0 535 565 B1**
(45) Veröffentlichungstag und Bekanntmachung des Hinweises auf die Patenterteilung: **13.12.1995**
(21) Anmeldenummer: 92116518.9
(22) Anmeldetag: 28.09.1992
(51) Int. Cl.: C07C 29/141, C07C 31/20

(54) **Verfahren zum Herstellen von 1,3-Propandiol durch Hydrieren von Hydroxypropionaldehyd**
Process for the preparation of 1,3-propanediol by hydrogenation of hydroxypropionaldehyde
Procédé de préparation du 1,3-propanediol par hydrogénation de la hydroxypropionaldéhyde

(30) Priorität: 01.10.1991 DE 4132663
(43) Veröffentlichungstag der Anmeldung: 07.04.1993
(73) Patentinhaber: Degussa Aktiengesellschaft, 60311 Frankfurt (DE)
(72) Erfinder: Arntz, Dietrich, Dr., W-6370 Oberursel (DE); Haas, Thomas, Dr., W-6090 Rüsselsheim (DE); Schäfer-Sindlinger, Adolf, Dr., W-6000 Frankfurt 60 (DE)

(56) Entgegenhaltungen:
- EP-A- 0 343 475
- EP-A- 0 412 337

## Beschreibung

Die vorliegende Erfindung betrifft ein Verfahren zum Herstellen von 1,3-Propandiol durch Hydrieren von Hydroxypropionaldehyd (HPA) in wäßriger Lösung an einem Festbettkatalysator.

1,3-Propandiol besitzt als Monomerbaustein für Polyester und Polyurethane sowie als Ausgangsstoff für die Synthese zyklischer Verbindungen vielseitige Anwendungsmöglichkeiten. Zur Herstellung von 1,3-Propandiol sind verschiedene Verfahren bekannt, die entweder von einem Molekülaufbau aus einem C₂ und C₁-Baustein oder von einem C₃-Baustein, wie z. B. Acrolein, ausgehen. Bei Verwendung von Acrolein wird dieses in Gegenwart eines sauren Katalysators zunächst hydratisiert, wobei Hydroxypropionaldehyd gebildet wird. Das bei der Hydratisierung gebildete wäßrige Reaktionsgemisch enthält nach Abtrennung von nicht umgesetztem Acrolein neben ca. 85 % HPA noch etwa 8 % Oxaheptandial und weitere organische Komponenten in geringeren Gewichtsanteilen. Zur Herstellung von 1,3-Propandiol wird dieses Reaktionsgemisch in Gegenwart von Hydrierkatalysatoren hydriert.

Gemäß US 2,434,110 sind für die Hydrierung von HPA zu 1,3-Propandiol Katalysatoren, welche ein oder mehrere hydrierwirksame Metalle, wie z. B. Fe, Co, Ni, Cu, Ag, Mo, W, V, Cr, Rh, Pd, Os, Ir, Pt enthalten, geeignet.

Wie in DE P 39 26 136.0 beschrieben kann der Katalysator sowohl in suspendierter Form per se oder trägergebunden vorliegen oder Bestandteil von Festbettkatalysatoren sein; auch homogene Katalysatoren können herangezogen werden. Als Suspensionskatalysatoren sind Raney-Nickel, das mit verschiedenen anderen katalytisch wirksamen Metallen dotiert sein kann, sowie Platin auf Aktivkohle und Platin auf Aluminiumoxid (aus US 3,536,763) bekannt. Eine hohe Raum-Zeit-Ausbeute wird bei der Hydrierung erzielt, wenn die zu hydrierende Lösung einen pH-Wert im Bereich von 2,5 bis 6,5 aufweist, die Hydriertemperatur im Bereich zwischen 30 und 180° C liegt und bei Wasserstoffdrucken von 5 bis 300 bar gearbeitet wird.

Hauptsächlich werden für die Hydrierung Nickel-Katalysatoren eingesetzt. Dabei werden Festbettkontakte bevorzugt, da sich bei ihnen ein Abfiltrieren des Katalysators nach erfolgter Hydrierung erübrigt. Ein typischer Festbettkontakt für diese Anwendung ist z. B. Nickel auf Al₂O₃/SiO₂.

Die katalytische Hydrierung birgt die Gefahr in sich, daß das katalytisch aktive Element in geringen Mengen in Form löslicher Verbindungen in den Produktstrom ausgetragen wird und weitere Arbeitsschritte zur Abtrennung dieser Verunreinigungen notwendig werden. Das ist besonders bei Suspensionskatalysatoren wie Raney-Nickel zu beobachten, aber auch bei den Nickel-Festbettkontakten besteht noch die Gefahr einer Verunreinigung des Produktes durch Nickelverbindungen, wenn auch in sehr geringen Mengen.

Hydrierverfahren können durch die mit ihnen erzielbaren Umsätze, Selektivitäten und Raum-Zeit-Ausbeuten charakterisiert werden. Der Umsatz gibt an, wieviel Mol des Eduktes (hier HPA) durch die Hydrierung in andere Stoffe umgesetzt werden. Die Angabe erfolgt gewöhnlich in Prozent der eingesetzten Mol des Eduktes. Die Selektivität des Hydrierverfahrens ist dagegen ein Maß dafür, wieviel Mol des Eduktes in das gewünschte Produkt überführt werden. Für kontinuierliche Hydrierverfahren ist die Raum-Zeit-Ausbeute eine weitere wichtige Kenngröße, die die erzielbare Menge des Produktes pro Zeiteinheit und Reaktorvolumen angibt.

Bei der großtechnischen Hydrierung von HPA zu 1,3-Propandiol ist es entscheidend für die Wirtschaftlichkeit des Hydrierverfahrens und für die Qualität des Produktes, daß Umsatz und Selektivität möglichst nahe 100 % liegen. Zwar wird nach der Hydrierung das Propandiol von dem im Produktstrom enthaltenem Wasser und restlichen HPA sowie Nebenprodukten durch Destillation getrennt, diese destillative Trennung wird jedoch durch Rest-HPA und Nebenprodukte wesentlich erschwert oder sogar durch Reaktionen zwischen Rest-HPA und Propandiol zu Acetalen, deren Siedepunkt nahe beim Siedepunkt von Propandiol liegt, völlig unmöglich gemacht. Je geringer Umsatz und Selektivität sind, umso schlechter ist also auch die erreichbare Produktqualität.

Umsatz, Selektivität und Raum-Zeit-Ausbeute werden durch die Eigenschaften des Katalysators und durch die Hydrierbedingungen wie Reaktionstemperatur, Wasserstoffdruck und Hydrierdauer oder im Falle kontinuierlicher Hydrierungen durch die Raumgeschwindigkeit LHSV (Liquid hourly space velocity) beeinflußt.

Bei der Hydrierung von HPA zu Propandiol ist zu beobachten, daß die Hauptreaktion linear vom Wasserstoff-Druck und der Zeit (Raumgeschwindigkeit bei kontinuierlichen Verfahren) abhängt, während die Reaktionstemperatur kaum Einfluß hat. Dagegen ist die Bildung von Nebenprodukten exponentiell von der Temperatur abhängig. Bei sonst gleichen Bedingungen ist eine Verdopplung der Bildung von Nebenprodukten pro 10° C Temperaturerhöhung zu beobachten, was zu einer entsprechenden Verschlechterung der Selektivität führt. Positiv auf die Selektivität wirkt sich dagegen die Erhöhung des Wasserstoff-Druckes aus, allerdings ist der postive Einfluß des Druckes auf die Selektivität weniger stark als der negative einer Temperaturerhöhung, da der Wasserstoff-Druck die Geschwindigkeit der Hauptreaktion nur linear, ein Temperaturanstieg die Geschwindigkeit der Nebenreaktion jedoch exponentiell erhöht.

Ein wesentliches Qualitätskriterium für die beim Hydrierverfahren eingesetzten Katalysatoren ist ihre Standzeit im Betrieb, d. h. gute Katalysatoren sollten im Laufe der Betriebszeit gleichbleibenden Umsatz und Selektivität bei der Hydrierung von HPA zu Propandiol gewährleisten. Hier zeigen Hydrierverfahren aus dem Stand der Technik insbesondere auf der Basis von Nickelkatalysatoren ungenügende Langzeit-Stabilitäten, was einen häufigeren Wechsel der gesamten Katalysatorpackung mit den bekannten Problemen bei der Entsorgung und Aufarbeitung von nickelhaltigen Verbindungen erfordert. Es ist deshalb Aufgabe der vorliegenden Erfindung, ein Hydrierverfahren anzugeben, das die erwähnten Nachteile von Verfahren aus dem Stand der Technik nicht aufweist und sich besonders durch ein verbessertes Langzeitverhalten auszeichnet.

Diese Aufgabe wird erfindungsgemäß durch ein Verfahren zum Herstellen von 1,3-Propandiol durch Hydrieren von Hydroxypropionaldehyd (HPA) in wäßriger Lösung an einem geformten Trägerkatalysator im Festbett gelöst, welches dadurch gekennzeichnet ist, daß der Trägerkatalysator aus Titanoxid besteht, auf dem Platin in einer Menge relativ zum Träger von 0,1 bis 5,0 Gew.-% in fein verteilter Form vorliegt.

In einer besonders gunstigen Ausführungsform der Erfindung wird als Titanoxid ein durch Flammenhydrolyse aus Titantetrachlorid gewonnenes sogenanntes pyrogenes Titanoxid mit einer BET-Oberfläche von 40 bis 60 m²/g und einem Gesamtporenvolumen von 0,25 bis 0,75 ml/g verwendet, das eine mittlere Größe der Primärteilchen von 20 nm, eine Dichte von 3,7 g/cm³ und eine Röntgenstruktur aus 20 bis 40 % Rutil und 80 bis 60 % Anatas aufweist und dessen Verunreinigungen an Siliciumdioxid, Aluminiumoxid und Eisenoxid unter 0,5 Gew.-% liegen. Pyrogenes Titanoxid wie das Material P25 von Degussa ist als Träger für die katalytisch aktive Komponente besonders geeignet. Es weist eine hohe spezifische Oberfläche nach BET von im Mittel 50 m²/g (gemessen nach DIN 66131) auf.

Das pyrogene Titanoxid wird zu Formkörpern wie z. B. Pellets, Granulat oder Strangpreßlingen verarbeitet und anschließend zweckmäßigerweise unter Verwendung einer löslichen Platinverbindung, vorzugsweise Hexachloroplatinsäure, mit der geforderten Menge Platin imprägniert, anschließend getrocknet und bei Temperaturen zwischen 250 und 500° C im Wasserstoffstrom über eine Dauer von 1 bis 10 h reduziert. Diese Präparation führt zu einer feinen Verteilung des Platins auf dem Katalysatorträger mit Kristallitgrößen von 1 bis 10 nm und einer Kohlenmonoxid-Adsorption von 0,5 bis 1,6 ml CO/g Katalysator. Fur die Imprägnierung sind auch andere Platinverbindungen wie z. B. Tetraaminplatin(II)-nitrat, Tetraaminplatin(II)-hydroxid oder Tetraaminplatin(II)-chlorid-1-hydrat geeignet.

Ein besonderer Vorteil des erfindungsgemäßen Verfahrens ist die gegenüber herkömmlichen Verfahren für die Hydrierung von 1,3-Propandiol verbesserte Standzeit des Katalysators im Betrieb. Darüberhinaus kann auch nach mehreren hundert Stunden Rieselbettbetrieb im Rahmen der Meßgenauigkeit keinerlei Platin-Austrag mit dem Produktstrom festgestellt werden. Das zeugt von einer sehr guten Fixierung des Platins auf dem Titanoxid-Träger.

Im folgenden wird die Erfindung anhand einiger Beispiels näher erläutert. Es zeigen
- Figur 1:: Langzeitverhalten eines erfindungsgemäßen Hydrierverfahrens mit Pt/TiO₂-Katalysator
- Figur 2:: Langzeitverhalten eines Vergleichsverfahrens mit Ni/Al₂O₃/SiO₂-Katalysator
In diesen Beispielen werden standardmäßige Ni/Al₂O₃/SiO₂-Katalysatoren mit den erfindungsgemäß einzusetzenden Katalysatoren bezüglich Umsatz und Selektivität im Batch-Betrieb und bezüglich ihres Langzeitverhaltens im Rieselbett miteinander verglichen.

Bei den zum Vergleich mit dem Stand der Technik herangezogenen Ni/Al₂O₃/SiO₂-Katalysatoren handelte es sich um den Typ "Girdler G134ARS" von Fa. Südchemie mit einem Gehalt von etwa 30 - 40 Gew.-% Nickeloxid. Zum Einsatz kamen Strangpreßlinge von 1,5 mm Durchmesser und Längen von 1 bis 3 mm.

Zur Charakterisierung der Katalysatorträger und der fertig imprägnierten Katalysatoren wurden nach dem Kalzinieren der Träger ihre spezifische BET-Oberfläche und ihre Porenradienverteilung bestimmt. Nach dem Imprägnieren der Träger mit geeigneten Platinverbindungen und Reduzieren dieser Verbindungen zu im wesentlichen metallischem Platin wurde die aktive Metalloberfläche durch CO-Adsorptionsmessungen ermittelt.

Die Bestimmung der spezifischen Oberfläche ist in DIN 66131 genormt. Bei den Poren wird zwischen Mikroporen mit Durchmessern kleiner als 2 nm, Mesoporen mit Durchmessern zwischen 2 und 50 nm und Makroporen mit Durchmessern größer als 50 nm unterschieden. Das Volumen der Mikroporen wird über die StickstoffAdsorption und Auswertung nach Brunauer, Emmett und Teller ermittelt. Für die Bestimmung der Mesoporen wird die Stickstoff-Adsorption nach De Boer ausgewertet. Die Makroporen werden mit Hilfe der Quecksilber-Porosometrie bestimmt.

Als Maß für die aktive Metalloberfläche der fertigen Katalysatoren dient die Kohlenmonoxid-Adsorption. Die Höhe dieses Wertes gibt Auskunft über die Güte der Metalldispersion. Durch eine zusätzliche transmissionselektronenmikroskopische (TEM) Untersuchung kann die Korngrößenverteilung der Metall-Kristallite direkt bestimmt werden.

### Präparation der Pt/TiO₂-Katalysatoren

Für die Herstellung der Katalysatorträger kam durch Flammenhydrolyse aus Titantetrachlorid gewonnenes sogenanntes pyrogenes Titanoxid der Firma Degussa (TiO₂-P25) zum Einsatz. Dieses Material hat eine spezifische Oberfläche von 48 m²/g und ein Gesamtporenvolumen von 0,33 ml/g, das sich auf 0,27 ml/g Mesoporen und 0,06 ml/g Makroporen aufteilt. Das pyrogene Titanoxid besteht zu ca. 30 % aus Rutil und zu ca. 70 % aus Anatas. Seine Primärteilchen haben eine mittlere Größe von etwa 20 nm.

Dieses Material kam zum einen direkt zum Einsatz und alternativ dazu nach mehrstündiger Temperung bei Temperaturen über 600° C. Durch die Temperung wandelte sich die Kristallstruktur des Materials vollständig in Rutil um. Die spezifische BET-Oberfläche verringerte sich auf Werte unter 13 m²/g. In den folgenden Beispielen wird das ungetemperte Material als pyrogenes Titanoxid (pyrog. TiO₂) und das getemperte Material als getempertes Titanoxid (getemp. TiO₂) bezeichnet.
A) Anfertigung von Granulat
   1000 g Titanoxid P25 der Fa. Degussa wurden im ungetemperten Zustand in einen Granulierbehälter (Eirich-Mischer, Fa. Eirich) gefüllt und unter langsamer Zugabe von insgesamt
   350 ml vollentsalztem Wasser granuliert. Während des Granuliervorgangs stieg die Temperatur im Mischer von Raumtemperatur auf 50° C an. Nach ca. 9 Minuten war die Granulierung beendet. Anschließend wurde das Granulat bei 200° C im Drehrohrofen getrocknet.
B) Anfertigung von Strangpreßlingen
   1000 g Titanoxid P25 der Fa. Degussa wurden im ungetemperten oder getemperten Zustand zusammen mit 5 l vollentsalztem Wasser in einem Knetextruder (Fa. Werner und Pfleiderer) vorgelegt und kurz geknetet bis Wasser und Titanoxid homogen vermischt waren. Dann wurden 10 g Tylose (MH 1000, Fluka) in 200 ml vollentsalztem Wasser gelöst hinzugefügt. Die Mischung wurde anschließend eine Stunde geknetet, bis eine plastische Masse entstanden war. Danach wurde die Masse zu Strangpreßlingen von 1,6 mm Durchmesser und ca. 10 mm Länge extrudiert und für ca. 12 Stunden in einem Trockenschrank bei 110° C getrocknet. Nach der Trocknung wurden die Formkörper in einem Heißluft-Temperofen bei 400° C für die Dauer von einer Stunde kalziniert.
   Die fertigen Strangpreßlinge und Granulate besaßen bei Verwendung von pyrogenem Titanoxid eine BET-Oberfläche von 48 m²/g und ein Gesamtporenvolumen von 0,33 ml/g, das sich auf 0,27 ml/g Mesoporen und 0,06 ml/g Makroporen aufteilte. Mikroporen konnten im Rahmen der Meßgenauigkeit nicht festgestellt werden. Durch die Verarbeitung des pyrogenen TiO₂-Ausgangsmaterials zu Formkörpern wurden also die Eigenschaften des Titanoxids wie spezifische Oberfläche und Porenvolumen kaum verändert. Die Formkörper aus getempertem Titanoxid besaßen nach der Formgebung ebenso wie das Ausgangsmaterial eine spezifische Oberfläche unter 13 m²/g.

Die so hergestellten Katalysator-Träger wurden nach der "Incipient-Wetness-Methode" imprägniert. Dazu wurde zunächst die maximale Wasseraufnahmefähigkeit der Träger bestimmt und anschließend eine Lösung aus Hexachloroplatinsäure angesetzt, deren Volumen ca. 95 % der maximalen Aufnahmefähigkeit der vorgelegten Menge der Katalystor-Träger entsprach und deren Platin-Gehalt auf den gewünschten Platin-Gehalt des fertigen Katalysators eingestellt wurde. Durch Zugabe von Salzsäure wurde der pH-Wert der Lösung auf den pH = 4 gebracht und die Lösung anschließend über die Katalysator-Träger gleichmäßig verteilt. Nachdem die gesamte Lösungsmenge aufgesogen war, wurden die imprägnierten Formkörper im Vakuumtrockenschrank zunächst bei 70° C und 150 mbar für eine Stunde vorgetrocknet und dann für weitere zwei Stunden bei 24 mbar fertiggetrocknet.

Nach dem Imprägnieren liegen in den Katalysator-Trägern homogen verteilte Kristalle aus Hexachloroplatinsäure vor. Sie wurden zur Aktivierung der Katalysatoren im Wasserstoffstrom zu metallischem Platin reduziert. Dazu wurden die imprägnierten Träger unter Stickstoffspülung auf 230° C aufgeheizt. Bei Erreichen von 230° C wurde auf Wasserstoffspülung umgeschaltet. Die einsetzende Reaktion zeigte eine deutliche Exotherme, so daß die Temperatur während der Reduktion auf ca. 280° C anstieg. Nach ca. einer Stunde wurde auf 100° C abgekühlt und so lange Stickstoff über den Katalysator geleitet, bis Raumtemperatur erreicht war.

An den fertigen Katalysatoren mit 2 Gew.-% Platin wurden ESCA- und SIMS-Untersuchungen durchgeführt. Sie zeigten, daß mit zunehmender Reduktionsdauer Platin als Pt(O), also in metallischer Form vorliegt. Der Anteil von Pt(II) geht von 25 % nach einstündiger Reduktion auf 14 % nach 10-stündiger Reduktion zurück. TEM-Untersuchungen ergaben eine homogene Verteilung von Platin-Kristallen mit Größen zwischen 2 und 5 nm. CO-Adsorptionsmessungen ergaben einen Wert von 1,66 ml CO/g Katalysator.

### Beispiel 1: Vergleich der Aktivitäten von Ni/Al₂O₃/SiO₂-Katalysatoren mit erfindungsgemäß einzusetzenden Pt-Katalysatoren auf pyrogenen TiO₂-Trägern bei der Batch-Hydrierung von HPA im Autoklaven

Es wurden Nickel-Festbettkatalysatoren (Girdler G134ARS von Südchemie) mit erfindungsgemäß zu verwendenden Pt-Katalysatoren auf pyrogenen TiO₂-Trägern bezüglich ihrer Aktivität bei der Batch-Hydrierung von wäßriger HPA-Lösung miteinander verglichen. Zum Vergleich kamen erfindungsgemäß zu verwendende Katalysatoren in Granulatform mit einer Edelmetallbeladung von 2 Gew.-% Platin auf pyrogenem Titanoxid zum Einsatz. Es wurden jeweils Umsatz und Selektivität bestimmt.

Im einzelnen wurden die Hydrierungen wie folgt vorgenommen: In einem 2 l Hastelloy-Autoklaven mit Begasungsrührung und Flüssigkeitsumwälzung wurden jeweils 23,5 g Katalysator entsprechend einem Substrat zu Katalysatorverhältnis von 400 : 1 in den vorbereiteten Katalysatorkorb eingewogen.

Anschließend wurde der Autoklav evakuiert, jeweils 750 ml HPA-Lösung mit einem bestimmten Mol-Gehalt HPA eingesaugt und Wasserstoffgas bis zu einem Druck von 150 bar eingelassen. Nach Einschalten der Rührung wurde zunächst während 15 Minuten auf 50° C aufgeheizt. Die einsetzende Reaktion zeigte eine leichte Exotherme, so daß die eigentliche Hydrierung bei etwa 60 - 65° C stattfand. Während der Reaktion wurde der Temperatur- und Druckverlauf alle 15 Minuten kontrolliert. Nach 4 Stunden wurden die Hydrierungen abgebrochen.

Tabelle 1 zeigt die nach dieser Zeit erzielten Werte für Umsatz und Selektivität. Spalte 4 enthält die in der wäßrigen Edukt-Lösung enthaltenen Mol HPA. 0,94 Mol HPA in 750 ml wäßriger Lösung entsprechen dabei einem Gewichtsanteil des HPA von ca. 10 %. In Spalte 5 ist die Menge des erzeugten 1,3 Propandiol in Mol eingetragen. Der aus dem Stand der Technik bekannte Nickel-Vergleichskatalysator (in Tabelle 1 mit "V" bezeichnet) setzt zwar das gesamte eingesetzte HPA um, seine Selektivität ist jedoch schlecht. Nur 74,5 bzw. 86,3 % der eingesetzten Mol HPA werden in 1,3-Propandiol überführt. Der erfindungsgemäß einzusetzende Katalysator auf einem pyrogenen TiO₂-Träger (in Tabelle 1 mit "K1" bezeichnet) weist dagegen eine hervorragende Selektivität auf.

**Tabelle 1**

| Kat | Metall | Träger | HPA (Mol) | 1,3-PD (Mol) | Umsatz ( % ) | Selektivität (%) |
|---|---|---|---|---|---|---|
| V | Ni | Al₂O₃/SiO₂ | 0,94 | 0,70 | 100 | 74,5 |
| V | Ni | Al₂O₃/SiO₂ | 0,73 | 0,63 | 100 | 86,3 |
| K1 | Pt(2%) | pyrog.TiO₂ | 0,94 | 0,88 | 97,9 | 95,6 |
| K1 | Pt(2%) | pyrog.TiO₂ | 0,77 | 0,76 | 100 | 98,7 |
| H₂-Druck: 150 bar; T = 60 - 65ó C; | | | | | | |

### Beispiel 2: Vergleich unterschiedlicher Pt-Gehalte auf pyrogenen TiO₂-Trägern

Zur Untersuchung der Abhängigkeit der katalytischen Aktivität der erfindungsgemäß zu verwendenden Katalysatoren vom Pt-Gehalt wurden die pyrogenen Titanoxid-Träger mit unterschiedlichen Platin-Beladungen hergestellt. Die Testhydrierungen im Hastelloy-Autoklaven entsprechend Beispiel 1 ergaben die in Tabelle 2 aufgeführten Meßwerte. Selektivität und Umsatz wachsen mit zunehmendem Platin-Gehalt an.

**Tabelle 2**

| Kat | Metall | Träger | HPA (Mol) | 1,3-PD (Mol) | Umsatz ( % ) | Selektivität (%) |
|---|---|---|---|---|---|---|
| K3 | Pt(0,5%) | pyrog.TiO₂ | 0,774 | 0,705 | 98,9 | 92,2 |
| K2 | Pt(1,0%) | pyrog.TiO₂ | 0,770 | 0,73 | 98,7 | 96,0 |
| K1 | Pt(2,0%) | pyrog.TiO₂ | 0,770 | 0,76 | 100,0 | 98,7 |
| H₂-Druck: 150 bar; T = 60 - 65ó C; | | | | | | |

### Beispiel 3: Vergleich unterschiedlicher Pt-Gehalte auf getemperten TiO₂-Trägern

Die Untersuchungen in Beispiel 3 wurden analog zu Beispiel 2 durchgeführt. Der einzige Unterschied bestand in der Verwendung von getempertem Titanoxid anstelle von pyrogenem Titanoxid. Die Ergebnisse sind in Tabelle 3 aufgelistet.

**Tabelle 3**

| Kat | Metall | Träger | HPA (Mol) | 1,3-PD (Mol) | Umsatz ( % ) | Selektivität (%) |
|---|---|---|---|---|---|---|
| K4 | Pt(0,5%) | getemp.TiO₂ | 0,508 | 0,477 | 99,8 | 94,1 |
| K5 | Pt(1,0%) | getemp.TiO₂ | 0,503 | 0,476 | 99,6 | 95,1 |
| K6 | Pt(2,0%) | getemp.TiO₂ | 0,508 | 0,475 | 99,2 | 94,3 |
| H₂-Druck: 150 bar; T = 60 - 65ó C; | | | | | | |

Die Katalysatoren K4 bis K6 auf der Basis von getempertem Titanoxid ergaben bei der Batch-Hydrierung ähnliche Ergebnisse wie die Katalysatoren K1 bis K3 auf der Basis von pyrogenem Titanoxid. Sie zeigten jedoch bei mehrfacher Anwendung eine deutliche Abnahme von Umsatz und Selektivität im Vergleich zu den Katalysatoren auf pyrogenen Trägern.

### Beispiel 4: Langzeitverhalten von Pt-Katalysatoren auf pyrogenen Titanoxidträgern im Rieselbett

Zur kontinuierlichen Hydrierung im Rieselbett wurde eine Rieselbettanlage mit 1,3 l Reaktorvolumen eingesetzt. Die Anlage bestand aus einer Flüssigkeitsvorlage, einer Vorheizstrecke, dem Festbettreaktor und einem Flüssigkeitsabscheider. Die Reaktortemperatur wurde über einen Wärmeträgerölkreislauf eingestellt. Druck und Wasserstoffstrom wurden elektronisch geregelt. Die wäßrige HPA-Lösung wurde vor der Vorheizstrecke dem Wasserstoffstrom mit einer Pumpe zudosiert und das Gemisch am Kopf des Reaktors aufgegeben (Rieselbettfahrweise). Nach Durchlaufen des Reaktors wurde das gebildete Produkt in regelmäßigen Abständen aus dem Abscheidegefäß entnommen und der Wasserstoff mit Hilfe eines Kompressors kontinuierlich rezykliert. Das Produkt wurde mit HPCL auf nicht umgesetztes HPA untersucht und das gebildete 1,3-Propandiol mit GC bestimmt.

Es wurden der Nickel-Vergleichskatalysator und ein erfindungsgemäß zu verwendender Platin-Katalysator auf pyrogenem Titanoxid mit 2 % Platin-Beladung in Form von Strangpreßlingen auf das Langzeitverhalten von Umsatz und Selektivität untersucht. Die Konzentration des HPA in der Eduktlösung betrug in beiden Fällen 11 Gew.-%.

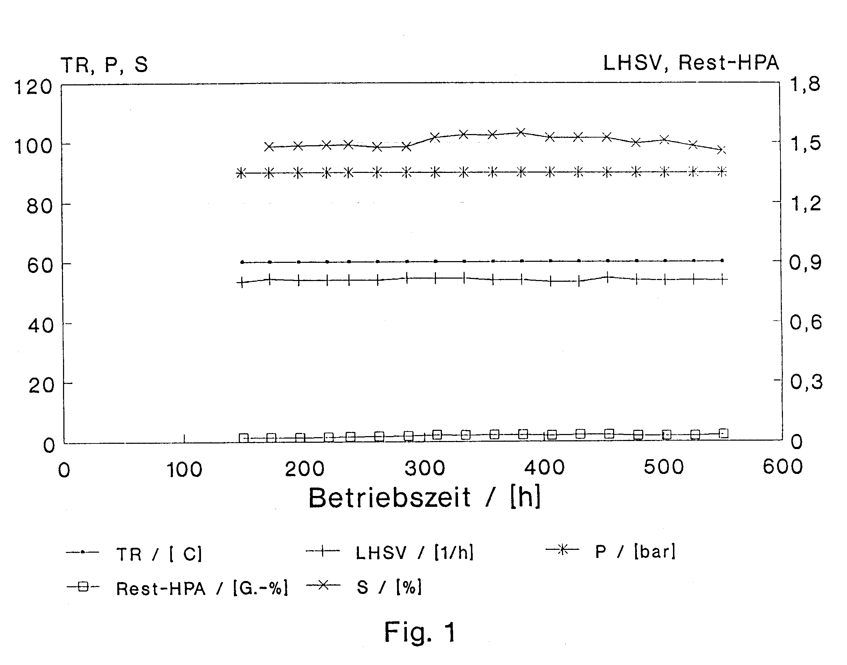

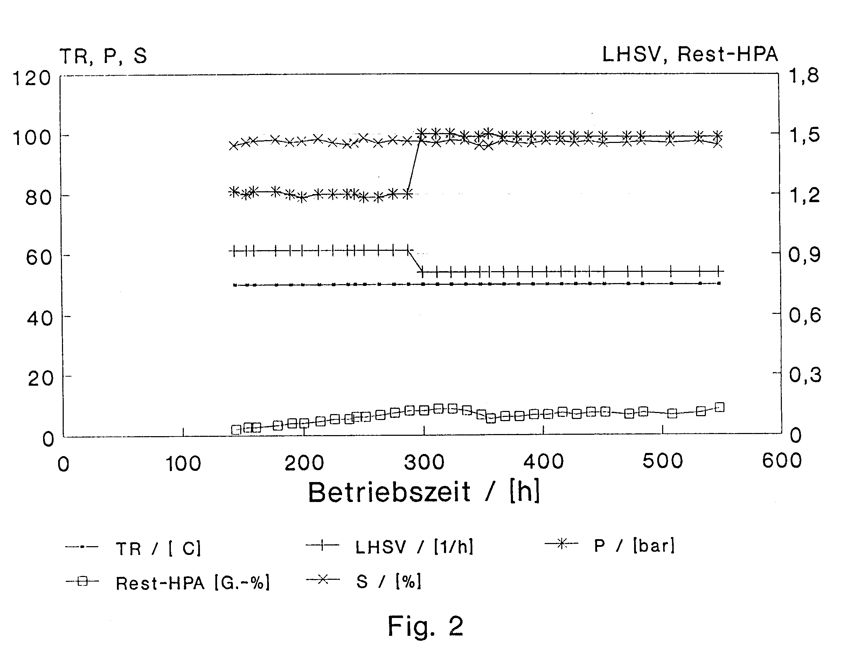

Die Figuren 1 und 2 zeigen den zeitlichen Verlauf von Reaktionstemperatur TR, Raumgeschwindigkeit LHSV (Liquid hourly space velocity), H₂-Druck P, Rest-HPA Gehalt der Produktlösung und die Selektivität S der Hydrierung in Abhängigkeit von der Betriebsdauer für das erfindungsgemäße Verfahren mit Pt/TiO₂-Katalysator (Fig. 1) und für das Vergleichsverfahren mit Nickel-Katalysator (Fig. 2). Wie den Diagrammen von Figur 1 und 2 zu entnehmen ist, wurde die Hydrierung mit dem Pt/TiO₂-Katalysator bei einer Reaktionstemperatur von 60° C, einem Wasserstoff-Druck von 90 bar und einer Raumgeschwindigkeit von 0,85 h⁻¹ durchgeführt, während beim Nickel-Katalysator eine Reaktionstemperatur von 50° C gewählt und anfänglich mit einem Wasserstoffdruck von 80 bar und einer Raumgeschwindigkeit von 0,9 h⁻¹ gearbeitet wurde.

Wegen der beschriebenen Verdopplung der Nebenproduktbildung pro 10° C Temperaturerhöhung waren die Hydrierbedingungen für den Pt/TiO₂-Katalysator ungünstiger. Trotzdem zeigt er einen hervorragend gleichbleibenden Umsatz. (Der Rest-HPA-Gehalt bleibt konstant.) Die Werte der Selektivität S liegen zum Teil über 100 %. Dieser Effekt ist durch die Tatsache zu erklären, daß die in der Eduktlösung enthaltenen organischen Beimengungen aus der Hydratisierung des Acroleins zum Teil ebenfalls zu 1,3-Propandiol umgesetzt werden.

Beim Nickel-Katalysator in Fig. 2 zeigt sich schon nach 200 Stunden Betriebszeit eine deutliche Zunahme des restlichen HPA. Nach ca. 290 Stunden war dieser Anteil so stark angewachsen, daß zu seiner Verminderung die Raumgeschwindigkeit von 0,9 h⁻¹ auf 0,85 h⁻¹ abgesenkt und der H₂-Druck auf 100 bar erhöht werden mußte.

## Patentansprüche

1. Verfahren zum Herstellen von 1,3-Propandiol durch Hydrierung von Hydroxypropionaldehyd (HPA) in wäßriger Lösung an einem geformten Trägerkatalysator im Festbett, wobei die Konzentration des HPA in der Lösung 5 bis 100 Gew.-% beträgt, und die Hydrierung bei Temperaturen von 30 bis 180° C und Wasserstoffdrucken von 5 bis 300 bar sowie bei einem pH-Wert zwischen 2,5 und 6,5 durchgeführt wird.
**dadurch gekennzeichnet**,
daß der Trägerkatalysator aus Titanoxid besteht, auf dem Platin in einer Menge relativ zum Träger von 0,1 bis 5,0 Gew.-% in fein verteilter Form vorliegt.

2. Verfahren nach Anspruch 1,
**dadurch gekennzeichnet**,
daß als Titanoxid ein durch Flammenhydrolyse aus Titantetrachlorid gewonnenes sogenanntes pyrogenes Titanoxid mit einer BET-Oberfläche von 40 bis 60 m²/g und einem Gesamtporenvolumen von 0,25 bis 0,75 ml/g verwendet wird, das eine mittlere Größe der Primärteilchen von 20 nm, eine Dichte von 3,7 g/cm³ und eine Röntgenstruktur aus 20 bis 40 % Rutil und 80 bis 60 % Anatas aufweist und dessen Verunreinigungen an Siliciumdioxid, Aluminiumoxid und Eisenoxid unter 0,5 Gew.-% liegen.

3. Verfahren nach Anspruch 2,
**dadurch gekennzeichnet**,
daß der Katalysatorträger unter Verwendung einer löslichen Platinverbindung, vorzugsweise Hexachloroplatinsäure, mit der geforderten Menge Platin imprägniert, anschließend getrocknet und bei Temperaturen zwischen 250 und 500° C im Wasserstoffstrom über eine Dauer von 1 bis 10 h reduziert wird.

4. Verfahren nach Anspruch 3,
**dadurch gekennzeichnet**,
daß das Platinmetall im Katalysatorträger fein verteilt mit Kristallitgrößen von 1 bis 10 nm vorliegt und seine Kohlenmonoxid-Adsorption 0,5 bis 1,6 ml Co/g Katalysator beträgt.

## Claims

1. Process for the preparation of 1,3-propanediol by hydrogenation of hydroxypropionaldehyde (HPA) in aqueous solution on a shaped supported catalyst in a fixed bed, with the concentration of the HPA in the solution being from 5 to 100% by weight and the hydrogenation being carried out at temperatures of from 30 to 180°C and hydrogen pressures of from 5 to 300 bar and at a pH value of between 2.5 and 6.5,
characterised in that
the supported catalyst consists of titanium oxide, whereon platinum is present in finely divided form in a quantity of from 0.1 to 5.0% by weight, relative to the support.

2. Process according to claim 1,
characterised in that
the titanium oxide used is a so-called pyrogenic titanium oxide obtained from titanium tetrachloride by flame hydrolysis and having a BET surface area of from 40 to 60 m²/g and a total pore volume of from 0.25 to 0.75 ml/g, which titanium oxide has primary particles of an average size of 20 nm, a density of 3.7 g/cm³ and an X-ray structure consisting of 20 to 40% of rutile and 80 to 60% of anatase and the impurities therein of silicon dioxide, aluminium oxide and iron oxide are less than 0.5% by weight.

3. Process according to claim 2,
characterised in that
the catalyst support is impregnated with the required quantity of platinum using a soluble platinum compound, preferably hexachloroplatinic acid, and is then dried and reduced in the stream of hydrogen over a period of from 1 to 10 hours at temperatures of between 250 and 500°C.

4. Process according to claim 3,
characterised in that
the platinum metal is present in finely divided form in the catalyst support, having crystallite sizes of from 1 to 10nm, and its carbon monoxide adsorption is from 0.5 to 1.6 ml CO/g of catalyst.

## Revendications

1. Procédé de préparation de 1,3-propanediol par hydrogénation d'aldéhyde hydroxypropionique (HPA) en solution aqueuse sur un catalyseur sur support dans le lit fixe, dans lequel la concentration du HPA dans la solution atteint 5 à 100 % en poids et l'hydrogénation est réalisée à des températures de 30 à 180°C et à des pressions d'hydrogène de 5 à 300 bars, ainsi qu'à une valeur de pH comprise entre 2,5 et 6,5,
caractérisé en ce que le catalyseur sur support se compose d'oxyde de titane, sur lequel existe sous forme finement divisée du platine en une quantité par rapport au support de 0,1 à 5,0 % en poids.

2. Procédé selon la revendication 1, caractérisé en ce qu'on utilise comme oxyde de titane, un oxyde de titane appelé pyrogéné obtenu par hydrolyse à la flamme de tétrachlorure de titane et ayant une surface BET de 40 à 60 m²/g et un volume total de pores de 0,25 à 0,75 ml/g, qui comporte une taille moyenne des particules primaires de 20 mm, une densité de 3,7 g/cm³ et une structure aux rayons X de 20 à 40 % de rutile et de 80 à 60 % d'anatase et dont les impuretés en dioxyde de silicium, oxyde d'aluminium et oxyde de fer se situent en dessous de 0,5 % en poids.

3. Procédé selon la revendication 2 caractérisé en ce que le support de catalyseur est imprégné d'une quantité exigée de platine en utilisant un composé de platine soluble, de préférence de l'acide hexochloroplatinique, ensuite il est séché et il est réduit à des températures entre 250 et 500°C dans le courant d'hydrogène pendant une durée de 1 à 10 heures.

4. Procédé selon la revendication 3, caractérisé en ce que le métal platine est présent dans le support de catalyseur finement divisé avec des tailles de cristallite de 1 à 10 nm et son adsorption de monooxyde de carbone est de 0,5 à 1,6 ml de CO/g de catalyseur.
